# EUROPÄISCHE PATENTSCHRIFT

(11) **EP 2 103 264 B1**
(45) Veröffentlichungstag und Bekanntmachung des Hinweises auf die Patenterteilung: **14.12.2011**
(21) Anmeldenummer: 09003714.4
(22) Anmeldetag: 14.03.2009
(51) Int. Cl.: A61B 17/28, A61B 17/00

(54) **Medizinisches Instrument**
Medical instrument
Instrument médical

(30) Priorität: 20.03.2008 DE 102008015418
(43) Veröffentlichungstag der Anmeldung: 23.09.2009
(73) Patentinhaber: Richard Wolf GmbH, 75438 Knittlingen (DE)
(72) Erfinder: Boebel, Manfred, 75245 Bauschlott (DE); Prestel, Stephan, 76287 Rheinstetten-Mörsch (DE); Brüstle, Sybille, 75447 Sternenfels (DE); Falk, Ernst, 75447 Sternenfels-Diefenbach (DE); Buess, Gerhard Fritz, Prof. Dr., 86949 Windach (DE)
(74) Vertreter: Hemmer, Arnd

(56) Entgegenhaltungen:
- GB-A- 2 284 242
- US-A- 5 620 415
- US-A1- 2005 096 694
- US-A1- 2007 276 430

## Beschreibung

Die Erfindung betrifft ein medizinisches Instrument, insbesondere ein medizinisches Instrument für die translumenale endoskopische Chirurgie über natürliche Körperöffnungen.

Beispielsweise bei endoskopischen Operationen im Abdomen über einen transvaginalen Zugang muss ein relativ englumiger langer Zugangstubus verwendet werden. Bedingt durch diesen englumigen Zugangstubus ist es schwierig, am Operationsort, wie beispielsweise der Gallenblase, die notwendigen Freiräume zur Retraktion, Präparation, Dissektion und Koagulation zu schaffen. Erschwert wird dies zusätzlich dadurch, dass das optische System und die Instrumente alle aus der gleichen Richtung kommen. Ein weiteres Problem ist, dass sich die Handhaben der einzelnen Instrumente gegenseitig stören.

Aus US 2007/0276430 A1 (Basis für den Oberbegriff des Anspruchs 1) ist ein endoskopisches Instrument bekannt, welches an seinem distalen Ende abwinkelbar ausgebildet ist und dar über hinaus im Bereich der Handhabe einen knick- bzw. winkelbaren Abschnitt aufweist. Durch die biegbare Ausgestaltung des Schaftes ist es schwer, dieses Instrument genau zu positionieren.

Es ist daher Aufgabe der Erfindung, ein medizinisches Instrument zu schaffen, welches bei der translumenalen endoskopischen Chirurgie über natürliche Körperöffnungen zum einen verbesserte Operationsmöglichkeiten schafft und zum anderen den Einsatz mehrerer Instrumente ermöglicht, ohne dass sich die Handhaben der Instrumente gegenseitig behindern.

Diese Aufgabe wird durch ein medizinisches Instrument mit den im Anspruch 1 angegebenen Merkmalen gelöst. Bevorzugte Ausführungsformen ergeben sich aus den Unteransprüchen, der nachfolgenden Beschreibung sowie den beigefügten Figuren.

Das erfindungsgemäße medizinische Instrument weist in bekannter Wiese an seinem proximalen Ende eine Handhabe auf. Von dieser Handhabe aus erstreckt sich distalwärts ein Schaft, an dessen distalen Ende ein bewegbares Maulteil, beispielsweise zum Schneiden oder Greifen angeordnet ist. Der Schaft ist vorzugsweise sehr lang ausgebildet, um den Einsatz des Instrumentes durch einen langen und englumigen Zugangstubus, beispielsweise einen mehr als 400 mm langen Tubus zu ermöglichen. Der Schaft muss dazu länger als der Tubus sein.

Der Schaft ist erfindungsgemäß starr, d.h. nicht in einer Richtung quer zu seiner Längsachse biegsam ausgebildet. Dies ermöglicht sehr einfach eine präzise Positionierung des Schaftes. In dem Schaft sind mindestens zwei Abwinkelbereiche ausgebildet, in denen sich die Erstreckungsrichtung des Schaftes ändert. D.h. in diesen Abwinkelbereichen knickt der Schaft ab oder ist gebogen, sodass die Längsachsen der an den Abwinkelbereich angrenzenden Schaftabschnitte zueinander gewinkelt verlaufen. Hierbei wird vorzugsweise ein Winkel zwischen 5 und 30 Grad gewählt. Aufgrund der starren Ausgestaltung des Schaftes handelt es sich hierbei um einen fest vorgegebenen Knick- oder Biegewinkel des Schaftes in den Abwinkelbereichen. Von den zwei Abwinkelbereichen ist der eine im proximalen Bereich des Schaftes und der zweite im distalen Bereich des Schaftes gelegen, wobei sich der Schaft zwischen den beiden Abwinkelbereichen vorzugsweise gerade erstreckt. Dabei erstreckt sich der Schaft weiter bevorzugt mit dem größten Teil seiner Länge zwischen den beiden Abwinkelbereichen. Dieser gerade Abschnitt zwischen den beiden Abwinkelbereichen ist derjenige Abschnitt, welcher bei der Operation durch den Zugangstubus verläuft. Die beiden Abwinkelbereiche sind dann jeweils außerhalb des Zugangstubus, einer an der distalen Seite und einer an der proximalen Seite des Zugangstubus gelegen. Es ist zu verstehen, dass beide Abwinkelbereiche denselben Biege- oder Knickwinkel, jedoch auch unterschiedliche Biege- oder Knickwinkel aufweisen können.

Erfindungsgemäß ist ferner ein Abschnitt des Schaftes, in welchem der distale Abwinkelbereich gelegen ist, relativ zu einem Abschnitt des Schaftes, in dem der proximale Abwinkelbereich gelegen ist, um die Längsachse des Schaftes drehbar. Durch diese Drehbarkeit kann das distale Ende des Schaftes quer zur Längsachse eines Zugangstubuses bzw. quer zur Erstreckungsöffnung des Schaftes zwischen den beiden Abwinkelbereichen verdreht bzw. verschwenkt werden. Auf diese Weise werden größere Freiräume im Operationsgebiet geschaffen, verglichen mit dem Einsatz sich gerade erstreckender Instrumente. So können beim Einsatz zweier derartiger Instrumenten deren distalen Enden bzw. die dort angeordneten Maulteile durch entsprechende Drehung der beiden distalen Enden der Instrumente voneinander entfernt und aufeinander zu bewegt werden. So kann beispielsweise mit einem Instrument, welches als Fasszange ausgebildet ist, die Gallenblase gehalten und durch Rotation des Schaftabschnittes mit dem proximalen Abwinkelbereich aus dem Operations-Feld geschwenkt werden, sodass sich ein gewünschter Zugang zur Gallenblase einstellt. Ein anderes Instrument, welches als Schneidinstrument ausgebildet ist, kann dann durch Rotation seines Abschnittes mit dem proximalen Abwinkelbereich in eine optimale Schneidposition gebracht werden. Die sich verändernde OP-Situation kann dabei eine Nachjustage des Zugangstubuses und der Instrumente erforderlich machen.

Darüber hinaus ist erfindungsgemäß das Maulteil relativ zu dem Schaft, um die Längsachse des Schaftes an dessen distalen Ende drehbar. Dadurch kann das Maulteil mit seiner Öffnung in eine gewünschte Winkellage gebracht werden, um Gewebe zu ergreifen oder zu schneiden. Das Maulteil dreht sich dabei um die Längsachse des Schaftabschnittes, welcher sich von dem distalen Abwinkelbereich weiter distalwärts erstreckt. Dieser Abschnitt ist vorzugsweise gerade ausgebildet, kann jedoch auch eine weitere Krümmung aufweisen.

Durch den proximalen Abwinkelbereich wird erreicht, dass die Handhabe gegenüber der Haupt-Erstreckungsrichtung des Schaftes, d. h. der Erstreckungsrichtung zwischen den beiden Abwinkelbereichen, abgewinkelt ist. Dies ermöglicht es, dass beim Einsatz mehrerer Instrumente in ein und demselben Zugangstubus deren Handhaben sich an der proximalen Seite des Zugangstubus in unterschiedliche Richtungen erstrecken können, sodass die Betätigung der einzelnen Handhaben nicht behindert wird.

Der Schaft weist vorzugsweise ein äußeres Schaftrohr auf und der zumindest eine distale Abwinkelbereich ist vorzugsweise durch einen Winkel oder eine Biegung in dem äußeren Schaftrohr definiert. D.h. das äußere Schaftrohr ist in dem Abwinkelbereich gewinkelt oder gebogen ausgebildet, sodass sich die an beiden Seiten des Abwinkelbereiches gelegenen Abschnitte des Schaftrohres gewinkelt zueinander erstrecken. Diese Abschnitte sind vorzugsweise beide jeweils gerade ausgebildet. Das äußere Schaftrohr ist in diesem Abwinkelbereich starr ausgebildet, so dass ein fester Winkel bzw. eine feste Krümmung vorgegeben ist.

Das äußere Schaftrohr ist vorzugsweise relativ zu einem proximalen Abschnitt des Schaftes drehbar. Diese Drehung erfolgt um die Längsachse des Schaftabschnittes zwischen den beiden Abwinkelbereichen, welcher sich vorzugsweise gerade erstreckt. Durch diese Drehung wird das distale Ende des Schaftes auf einer Kreisbahn bewegt, welche radial beabstandet von der Drehachse ist. Auf diese Weise kann das distale Ende mit dem Maulteil in einer Richtung quer zur Haupt-Erstreckungsrichtung des Schaftes, d. h. der Erstreckungsrichtung zwischen den beiden Abwinkelbereichen, in seiner Position verlagert werden. Die Längsachse zwischen den Abwinkelbereichen erstreckt sich bei der Operation im Wesentlichen parallel zur Längsachse des Zugangstubus, da dies der Abschnitt des Schaftes ist, der im Zugangstubus gelegen ist.

Ferner weist der Schaft bevorzugt ein an die Handhabe angrenzendes, sich nur über einen proximalen Abschnitt des Schaftes erstreckendes starres Außenrohr auf, welches das äußere Schaftrohr außen umfänglich umgibt und den zumindest einen proximalen Abwinkelbereich durch einen Winkel oder eine Biegung in dem Außenrohr definiert. D.h. das starre Außenrohr ist gewinkelt oder gebogen und bildet somit den proximalen Abwinkelbereich mit einem festen Biege- bzw. Knickwinkel. So erstrecken sich die beiden an den Abwinkelbereich angrenzenden Abschnitte des Außenrohres mit ihren Längsachsen gewinkelt zueinander. Diese Abschnitte des Außenrohres sind vorzugsweise gerade ausgebildet. In proximale Richtung erstreckt sich das Außenrohr von dem Abwinkelbereich bis zur Handhabe und ist mit dieser verbunden. In distaler Richtung erstreckt sich das Außenrohr so weit, dass es in dem Schaftabschnitt zwischen den beiden Abwinkelbereichen endet. Vorzugsweise erstreckt sich das Außenrohr in distaler Richtung verglichen mit der Gesamtlänge des Schaftes nur geringfügig über den proximalen Abwinkelbereich hinaus.

Das äußere Schaftrohr ist bevorzugt relativ zu dem Außenrohr um seine Längsachse drehbar. Auf diese Weise werden zwei zueinander verdrehbare Abschnitte des Schaftes geschaffen. Der eine Abschnitt ist der durch das Außenrohr definierte Abschnitt mit dem proximalen Abwinkelbereich und der zweite Abschnitt ist der von dem äußeren Schaftrohr definierte Abschnitt mit dem distalen Abwinkelbereich.

Zur Drehung des äußeren Schaftrohres ist dieses vorzugsweise am proximalen Ende mit einer Dreheinrichtung in der Handhabe verbunden. Diese kann beispielsweise ein Drehrad sein, mit welchem das äußere Schaftrohr um seine Längsachse gedreht werden kann.

Um diese Drehbewegung von der Handhabe über den ersten Abbiegebereich hinaus übertragen zu können, ist das äußere Schaftrohr in dem proximalen Abwinkelbereich bevorzugt biegsam ausgebildet. Dazu kann das Schaftrohr in diesem Bereich beispielsweise aus mehreren beweglich miteinander verbundenen starren Rohrabschnitten ausgebildet sein. Ein geeigneter Schaft aus beweglich miteinander verbundenen Rohrabschnitten ist beispielsweise aus EP 0 764 423 B1 bekannt. Ein solch biegsam ausgebildeter Schaft kann sich auch in dem Abbiegebereich im Inneren des Außenrohres um seine Längsachse drehen. In distaler Richtung hinter dem Abbiegebereich ist das äußere Schaftrohr dann wieder starr, d.h. nicht biegbar ausgebildet. Dabei überlappen sich das starre Außenrohr und der starre Abschnitt des äußeren Schaftrohres über eine gewisse Länge, um eine stabile Führung für das äußere Schaftrohr in dem Außenrohr zu schaffen. Das äußere Schaftrohr ist vorzugsweise am Innenumfang des Außenrohres geführt bzw. gelagert. Insbesondere sollte der Anlagebereich zwischen starrem Teil des äußeren Schaftrohres und Außenrohr so lang gewählt sein, dass Querkräfte, welche auf das äußere Schaftrohr wirken, sicher auf das starre Außenrohr übertragen werden können. Distalseitig des distalen Endes des Außenrohres bildet das äußere Schaftrohr das dem Schaft seine Stabilität und Biegefestigkeit gebende Bauteil.

Weiter bevorzugt ist an dem distalen Ende des äußeren Schaftrohres das Maulteil drehbar angebracht und in dem äußeren Schaftrohr ist ein drehbares Innenrohr angeordnet, welches an seinem proximalen Ende mit einer Dreheinrichtung in der Handhabe und am distalen Ende drehfest mit dem Maulteil verbunden ist. Über diesen Innenschaft kann das Maulteil, wie oben beschrieben um seine Längsachse gedreht werden. Dazu wird die Dreheinrichtung, welche beispielsweise als Drehrad ausgebildet ist, gedreht. Damit dreht sich das Innenrohr um seine Längsachse konzentrisch zu dem umgebenden äußeren Schaftrohr und dem Außenrohr. Aufgrund einer drehfesten Verbindung zwischen Maulteil und Innenschaft dreht sich das Maulteil dann gemeinsam mit dem Innenschaft.

Das Drehrad für den Innenschaft und ein Drehrad für das äußere Schaftrohr sind in der Handhabe vorzugsweise so angeordnet, dass sie um dieselbe Drehachse drehbar sind und lediglich axial voneinander beabstandet sind. So können die beiden Handräder gleichzeitig ergriffen und unterschiedlich verdreht werden, sodass zeitgleich mit der Drehung des äußeren Schaftrohres auch das Maulteil verdreht werden kann, wobei Drehwinkel und Drehrichtung der beiden Elemente unabhängig voneinander sind.

Das Innenrohr ist zweckmäßigerweise zumindest in den Abwinkelbereichen biegsam ausgebildet. Dies kann beispielsweise dadurch geschaffen werden, dass das Innenrohr in diesen Bereichen aus mehreren beweglich miteinander verbundenen starren Rohrabschnitten ausgebildet ist, wie es aus EP 0 764 423 B1 bekannt ist. Durch die Biegsamkeit, d.h. eine Beweglichkeit quer zur Längsrichtung des Innenrohes wird sichergestellt, dass das Innenrohr über die gesamte Länge um seine Längsachse gedreht werden kann, durch die Abwinkelbereiche hindurch. D.h. das Innenrohr dreht sich in allen Bereichen des Schaftes um seine Längsachse konzentrisch zu dem umgebenden äußeren Schaftrohr. In den axialen Abschnitten des Innenrohres, in denen das umgebende äußere Schaftrohr sich gerade erstreckt, ist vorzugsweise auch das Innenrohr starr ausgebildet, da auf diese Weise eine reibungsärmere Bewegung des Innenrohres möglich ist und dem Schaft eine größere Stabilität gegeben wird. Ferner kann in diesen Bereichen auf die Bearbeitung zur Ausgestaltung der zueinander beweglichen Abschnitte kostensparend verzichtet werden.

Die Ausbildung des Innenrohres nach dem aus EP 0 764 423 B1 bekannten System bietet sich insbesondere dann an, wenn Abschnitte des Innenrohres, wie vorangehend beschrieben, nicht biegsam ausgebildet werden sollen. Nach dem aus EP 0 764 423 B1 bekannten Verfahren ist es nämlich sehr leicht möglich, nur einzelne Abschnitte eines Rohres biegsam auszubilden, indem dort die entsprechenden Trennfugen eingebracht werden. Dasselbe gilt für die Ausgestaltung des äußeren Schaftrohres. Auch hier bietet sich das aus EP 0 764 423 B1 bekannte Verfahren an, da sehr leicht die für die Beweglichkeit erforderlichen Trennfugen nur in einen axialen Abschnitt des Schaftrohres eingebracht werden müssen, nämlich in dem Abschnitt, welcher sich durch den Abwinkelbereich erstreckt.

Weiter bevorzugt erstreckt sich im Inneren des Schaftes von der Handhabe zu dem Maulteil eine Betätigungsstange zur Betätigung des Maulteils. Diese Betätigungsstange erstreckt sich durch das Innere des Innenrohres vom proximalen zum distalen Ende des Schaftes. Die Betätigung des Maulteiles erfolgt vorzugsweise durch axiales Verschieben der Betätigungsstange entlang der Längsachse des Schaftes. Dabei kann je nach Ausgestaltung des Instrumentes beispielsweise das Maulteil durch Ziehen der Betätigungsstange in proximaler Richtung geöffnet werden. Alternativ ist es auch möglich eine umgekehrte Bewegung vorzusehen, bei welcher das Maulteil durch Ziehen der Betätigungsstange in distaler Richtung geschlossen wird. Dies hängt insbesondere davon ab, bei welcher Betätigung die größere Kraft erforderlich ist. Ist zum Schließen des Maulteils die größere Kraft erforderlich, so bietet es sich an, die Betätigungsstange hierzu in proximaler Richtung zu ziehen. Damit die Betätigungstange die Abwinkelbereiche durchlaufen kann, ist sie zweckmäßigerweise ebenfalls biegsam ausgebildet, muss jedoch eine ausreichende Festigkeit aufweisen, um die erforderliche Druck- und/oder Zugkraft in axialer Richtung übertragen zu können.

Gemäß einer weiteren bevorzugten Ausführungsform ist das Maulteil lösbar mit dem äußeren Schaftrohr verbunden. Dies kann zur Reinigung oder zum Austausch des Maulteils zweckmäßig sein. Die lösbare Verbindung kann beispielsweise durch einen Gewindeeingriff oder durch einen Bajonettverschluss geschaffen werden. Das Maulteil weist vorteilsweise in axialer Richtung zwei gegeneinander verdrehbare Abschnitte auf. Der proximalseitig gelegene feste Abschnitt kann über ein Verbindungsmittel, beispielsweise ein Gewinde oder ein Bajonett lösbar mit dem distalen Ende des äußeren Schaftrohres verbunden werden. Dabei wird eine drehfeste Verbindung geschaffen. Gleichzeitig tritt durch geeignete Mittel zweckmäßigerweise das distale Ende des Innenrohres mit dem drehbaren Abschnitt des Maulteils kraft- und/oder formschlüssig in Eingriff, sodass zwischen beiden eine drehfeste Verbindung geschaffen wird. Hierzu können an dem drehbaren distalen Abschnitt des Maulteils und/oder am distalen Ende des Innenrohres geeignete Eingriffselemente und/oder Mitnehmer vorgesehen sein, welche miteinander in Eingriff treten.

Die Betätigungsstange ist distalseitig vorzugsweise fest mit dem Maulteil verbunden, d.h. insbesondere mit der im distalen drehbaren Abschnitt des Maulteils gelegenen Mechanik zum Öffnen und Schließen. Wenn das Maulteil von dem äußeren Schaftrohr abgenommen wird, wird dabei bei dieser Ausgestaltung dann die Betätigungsstange in distaler Richtung aus dem Innenrohr herausgezogen.

Gemäß einer weiteren bevorzugten Ausführungsform ist der Schaft lösbar mit der Handhabe verbunden. Diese Verbindung ist vorzugsweise wie in DE 103 57 105 B3 beschrieben ausgestaltet. Die lösbare Verbindung ist dabei vorzugsweise so ausgestaltet, dass sowohl Außenrohr, äußeres Schaftrohr, Innenrohr und Betätigungsstange von der Handhabe getrennt werden können, wobei die Trennung vorzugsweise in einem Arbeitsschritt automatisch erfolgt, d.h. die einzelnen Elemente nicht manuell einzeln voneinander getrennt werden müssen. Umgekehrt ist die Verbindung so ausgebildet, dass beim Zusammensetzen die Teile automatisch in der gewünschten Weise miteinander in Eingriff treten. Insbesondere sind dazu in der Handhabe Verbindungselemente vorgesehen, welche eine drehfeste Verbindung zwischen dem äußeren Schaftrohr und dem zugehörigen Handrad und zwischen dem Innenrohr und dem zugehörigen Handrad in dem Griffteil herstellen, wenn der Schaft mit dem Griffteil verbunden wird.

Gemäß einer besonderen Ausführungsform kann das Instrument als HF-Instrument ausgebildet sein. Dazu kann der das äußere Schaftrohr nach außen isoliert sein und an der Handhabe ein Anschluss für eine Hochfrequenz-Spannungsversorgung vorgesehen sein. Bevorzugt wird das Instrument dabei unipolar ausgebildet. Grundsätzlich ist jedoch auch eine bipolare Ausgestaltung denkbar, wobei dabei beispielsweise das äußere Schaftrohr und das Innenrohr und/oder die Betätigungsstange die Funktion der zwei erforderlichen elektrischen Leiter übernehmen können. Die Elemente müssen dann entsprechend elektrisch gegeneinander isoliert werden.

Nachfolgend wird die Erfindung beispielhaft anhand der beigefügten Figuren beschrieben. In diesen zeigt:
- Fig. 1: eine schematische Gesamtansicht eines medizinischen Instrumentes gemäß der Erfindung,
- Fig. 2: eine schematische Gesamtansicht eines Instrumentes gemäß einer zweiten Ausführungsform der Erfindung,
- Fig. 3: das Instrument gemäß Fig. 2 in teilweise geschnittener An- sicht,

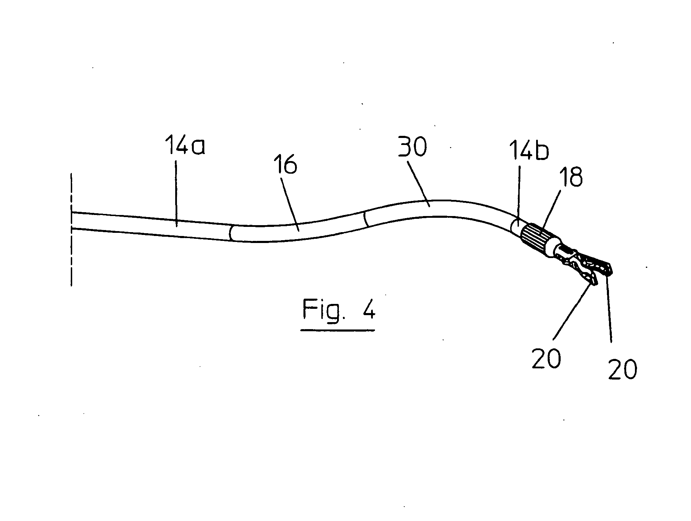

- Fig. 4: eine Detailansicht des distalen Endes des Instrumentes gemäß Fig. 2,
- Fig. 5: eine Schnittansicht entlang der Linie V - V in Fig. 2,
- Fig. 6: eine Einzelansicht des Maulteiles, des medizinischen In- strumentes gemäß Figuren 1 bis 5,
- Fig. 7: das Instrument gemäß Fig. 1, bei welchem der Schaft von der Handhabe getrennt ist,
- Fig. 8: das Instrument gemäß Figuren 1 und 7 mit teilweise ent- nommenen Maulteil, welches als Fasszange ausgebildet ist und
- Fig. 9: ein Instrument entsprechend Fig. 8, wobei das Maulteil als Schere ausgebildet ist.

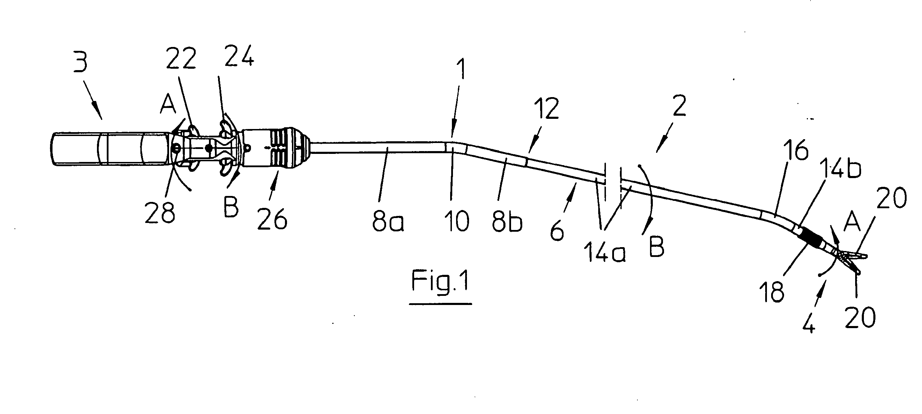

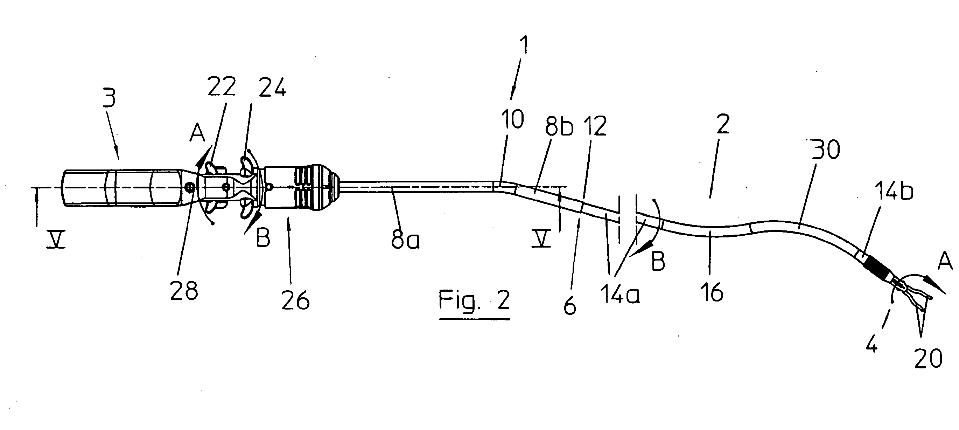

In beiden Ausführungsformen der Erfindung, d.h. der Ausführungsform gemäß Fig. 1 und der Ausführungsform gemäß Fig. 2 besteht das Instrument 1 grundsätzlich aus zwei Teilen, nämlich einem Zangeneinsatz 2 und einem Handhabenteil 3. Am distalen Ende des Zangeneinsatzes 2 ist ein Maulteil 4 ausgebildet. Ausgehend von dem Handhabenteil 3 erstreckt sich in distaler Richtung ein Schaft 6 zu dem Maulteil 4.

Dieser Schaft 6 ist aus mehreren Teilen ausgebildet. Direkt an die Handhabe 3 schließt sich in distaler Richtung zunächst ein starres Außenrohr 8 an, welches aus zwei zueinander gewinkelten Abschnitten 8a und 8b gebildet ist, welche über einen Abwinkelbereich 10 fest miteinander verbunden sind. Der Abschnitt 8a und der Abschnitt 8b sowie der Abbiegebereich 10 sind vorzugsweise einteilig oder einstückig ausgebildet. Aufgrund der starren Ausgestaltung des Außenrohres gibt der Abwinkelbereich 10 einen definierten Winkel der Längsachsen der Abschnitte 8a und 8b zueinander vor. Am distalen Ende 12 erstreckt sich aus dem Außenrohr 8 heraus ein äußeres Schaftrohr 14, welches ebenfalls aus zwei Abschnitten 14a und 14b gebildet ist, die über einen starren Abwinkelbereich 16 fest miteinander verbunden sind. Die Abschnitte 14a und 14b sind dabei mit dem Abwinkelbereich 16 einteilig oder einstückig ausgebildet. Aufgrund der starren Ausgestaltung des äußeren Schaftrohres 14 ist durch den definierten Winkel des Abwinkelbereiches 16 ein definierter Winkel zwischen den Längsachsen der Abschnitte 14a und 14b zueinander vorgegeben.

Insgesamt ist somit der Schaft zweimal gewinkelt, nämlich in dem Abwinkelbereich 10 und dem Abwinkelbereich 16. Der Abschnitt 14a des äußeren Schaftrohres 14 bildet den längsten Abschnitt des Schaftes 6 und ist derjenige Abschnitt, welcher bei der Operation im Inneren eines Zugangstubus gelegen ist. Der Abwinkelbereich 10 ist bei dieser Positionierung proximalseitig des Zugangstubuses gelegen, während der Abwinkelbereich 16 distalseitig des Zugangstubus gelegen ist. Am distalen Ende des Abschnittes 14b des äußeren Schaftrohres 14 ist mit diesem drehfest das Maulteil 4 durch die Gewindehülse 18 des Maulteiles verbunden. Anstelle der Gewindehülse kann auch ein Bajonettverschlussvorgesehen werden. Relativ drehbar zu dieser proximalen Gewindehülse 18 ist ein distaler Abschnitt des Maulteils 4, welcher im Wesentlichen von den beiden zueinander beweglichen Teilen 20 gebildet wird. Bei der Ausführungsform gemäß Fig. 1 sind die beweglichen Teile 20 des Maulteils als Schere ausgebildet. Diese Ausgestaltung und insbesondere die schwenkbewegliche Bewegbarkeit der Teile 20 zueinander kann in bekannter Weise erfolgen. Die Drehung relativ zu der proximalen Gewindehülse 18 erfolgt um die Längsachse des Abschnittes 14b des äußeren Schaftrohres 14.

Ferner ist das äußere Schaftrohr 14 um die Längsachse des Abschnittes 14a relativ zu dem Außenrohr 10 drehbar. Die Drehung des Maulteiles 4 und des äußeren Schaftrohres 14 erfolgt durch zwei Handräder 22 und 24 in dem Handhabenteil 3. Dabei ist das Handrad 22 für die Drehung des Maulteils 4 und das Handrad 24 für die Drehung des äußeren Schaftrohres 14 vorgesehen. Das bedeutet, wenn das Handrad 22 in Richtung des Pfeils A gedreht wird, dreht sich entsprechend das Maulteil 4 in Richtung des Pfeils A. Wen das Handrad 24 in Richtung des Pfeils B gedreht wird, dreht sich entsprechend das äußere Schaftrohr 14 in Richtung des Pfeils B (Fig. 1 - 3).

Der Zangeneinsatz 2 ist mit dem Handhabenteil 3 lösbar über eine Kupplung 26 verbunden. Diese Kupplung ist in der Weise ausgestaltet, wie es aus DE 103 57 105 B3 bekannt ist. Insbesondere sorgt die Kupplung 26 dafür, dass das Außenrohr 8 drehfest in einem vorbestimmten Winkel mit dem Handhabenteil 3 verbunden wird. Die Kupplung 26 stellt darüber hinaus eine lösbare Verbindung der weiteren in dem Außenrohr 10 gelegenen Rohre bzw. Schaftteile mit entsprechenden Komponenten in dem Handhabenteil 3 sicher. So wird das äußere Schaftrohr 14, welches sich in proximaler Richtung durch das Innere des Außenrohres 8 hindurch erstreckt, lösbar mit dem Handrad 24 gekoppelt. Entsprechend wird ein später näher beschriebenes Innenrohr zur Drehung des Maulteiles 4 mit dem Handrad 22 lösbar gekoppelt. Darüber hinaus wird eine Betätigungsstange zum Öffnen und Schließen der beweglichen Teile des Maulteils 20 lösbar mit einem Griffteil der Handhabe 3 gekoppelt.

Das Handhabenteil weist ferner einen HF-Anschluss 28 zur Verbindung mit einer Hochfrequenzspannungsquelle auf.

Die Ausführungsform gemäß Fig. 2 unterscheidet sich in zwei Punkten von der Ausführungsform gemäß Fig. 1. Zum einen sind die beweglichen Teile 20 des Maulteils bei der Ausführungsform gemäß Fig. 2 nicht als Schere, sondern als Fasszange ausgebildet. Zum anderen weist das äußere Schaftrohr 14 neben dem Abwinkelbereich 16 einen sich distalwärts an diesen anschließenden weiteren Abwinkelbereich 30 auf, in welchem das äußere Schaftrohr 14 noch einmal abgewinkelt wird, sodass der Abschnitt 14b des äußeren Schaftrohres 14 gegenüber dem Abschnitt 14a des äußeren Schaftrohres 14 zum einen gewinkelt und zum anderen um ein bestimmtes Maß querversetzt ist. Hierdurch kann eine andere Positionierung des Maulteils 4 im Operationsgebiet erreicht werden.

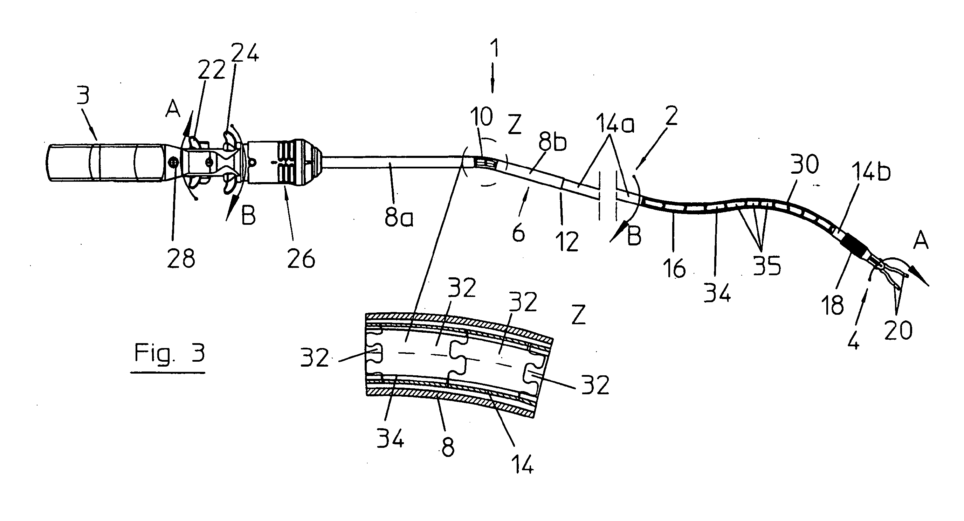

Anhand von Fig. 3 wird nun der innere Aufbau des Schaftes 6 näher erläutert, wobei zu verstehen ist, dass der innere Aufbau des Schaftes 6 bei der Ausführungsform gemäß Fig. 1 identisch zu dem in den Fig. 3 gezeigten Aufbau ist. Das äußere Schaftrohr 14 erstreckt sich von der Handhabe durch das Außenrohr 8 hindurch und tritt an dessen distalen Ende 12 nach außen aus. Damit dabei das äußere Schaftrohr 14 den Abwinkelbereich so durchlaufen kann, dass es um seine Längsachse drehbar ist, ist das äußere Schaftrohr 14 im Bereich des Abwinkelbereiches 10 biegsam ausgebildet. Dies ist in der Ausschnittsvergrößerung Z in Fig. 3 näher gezeigt. Wie zu erkennen ist, ist in diesem Bereich das äußere Schaftrohr 14 aus mehreren Rohrabschnitten 32 gebildet, welche über Eingriffselemente formschlüssig miteinander in Eingriff sind. Dabei ist der Eingriff so, dass die einzelnen Rohrabschnitt 32 relativ zueinander beweglich sind. Diese Ausgestaltung entspricht in ihrem Aufbau dem aus EP 0 764 423 B1 bekannten abwinkelbaren Rohr. Das in diesem europäischen Patent beschriebene Verfahren ermöglicht es, in das sonst starre äußere Schaftrohr 14 nur in dem Abwinkelbereich 10 entsprechende Trennfugen einzubringen, welche die zueinander beweglichen Rohrabschnitte 32 ausbilden.

im Inneren des äußeren Schaftrohres 14 ist ein Innenrohr 34 angeordnet, welches sich vom distalen Ende des Instrumentes 1 bis zu dem Handhabenteil 3 erstreckt. Das Innenrohr 34 ist im Inneren des äußeren Schaftrohres 14 um seine Längsachse drehbar. An seinem proximalen Ende ist das Innenrohr 34 mit dem Handrad 22 verbunden. Am distalen Ende ist das Innenrohr 34 mit dem drehbaren Abschnitt des Maulteiles 4 verbunden. Wenn das Handrad 22 in Richtung des Pfeils A gedreht wird, wird das Innenrohr 34 um seine Längsachse gedreht und überträgt diese Drehbewegung auf das Maulteil 4, sodass dessen distaler Abschnitt mit den beweglichen Teilen 20 in Richtung des Pfeils A gedreht wird. Um die Abwinkelbereiche 10, 16 und 30 entsprechend gewinkelt und dennoch um die Längsachse drehbar durchlaufen zu können, ist das Innenrohr 34 in diesen Bereichen biegbar ausgebildet. Die Ausgestaltung entspricht dabei der biegbaren Ausgestaltung des äußeren Schaftrohres 14 in dem Abwinkelbereich 10. D.h. in den gewinkelten bzw. gekrümmten Abschnitten ist auch das Innenrohr 34 aus einzelnen beweglich miteinander verbundenen Rohrabschnitten 35 gebildet, wie es aus EP 0 764 423 B1 bekannt ist. In der Vergrößerung Z ist diese Ausgestaltung des Innenrohrs 34 nicht im Einzelnen gezeigt, es ist jedoch zu verstehen, dass dort das Innenrohr 34 genauso ausgebildet ist, wie in den Abwinkelbereichen 16 und 30.

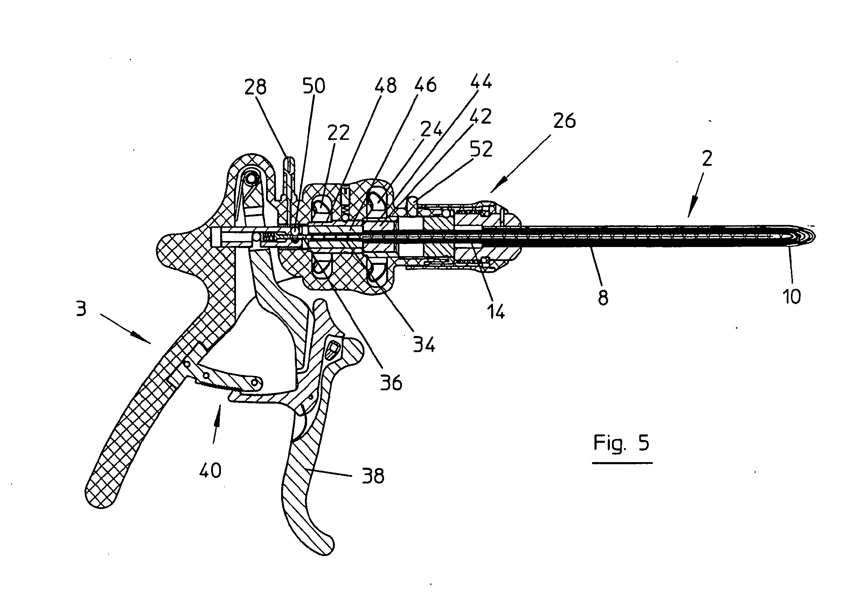

Wie in Figuren 5, 6 und 7 zu erkennen ist, erstreckt sich durch das Innere des Innenrohres 34 von dem Handhabenteil 3 zu dem Maulteil 4 eine Betätigungsstange 36, durch deren axiales Verschieben die beweglichen Teile 20 des Maulteils geöffnet und geschlossen werden können. Dazu ist das proximale Ende der Betätigungsstange 36 in dem Handhabenteil 3 mit einem beweglichen Handhabenteil 38 verbunden. Das bewegliche Handhabenteil 38 kann über eine Rastsperre 40 in seiner Position gesichert werden.

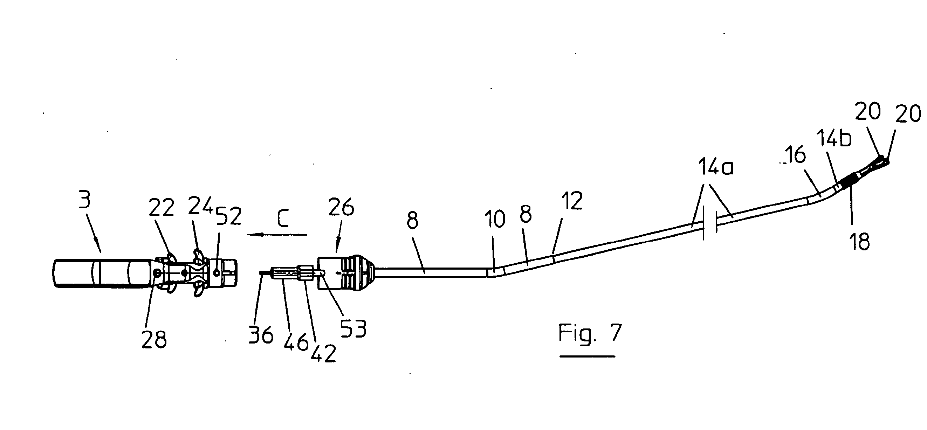

Wie in Figuren 5 und 7 zu erkennen ist, ist am proximalen Ende des äußeren Schaftrohres 14 ein Vielkant 42 drehfest angeordnet, welcher in einer korrespondierende Aufnahme 44 in dem Handrad 24 formschlüssig eingreift, um eine Drehbewegung von dem Handrad 24 über den Vielkant 42 auf das äußere Schaftrohr 14 zu übertragen. Entsprechend ist am proximalen Ende des Innenrohres 34 ein Vielkant 46 drehfest angeordnet, welcher formschlüssig in eine korrespondierende Aufnahme 48 in dem Handrad 22 eingreift. So kann eine Drehbewegung von dem Handrad 22 über den Vielkant 46 auf das Innenrohr 34 übertragen werden.

Diese Verbindung der Elemente ermöglicht es, dass das Kupplungsteil 26 in Richtung des Pfeils C mit dem Handhabenteil 3 zusammengesteckt werden kann. Wobei die Vielkante 42 und 46 mit den korrespondierenden Aufnahmen 44 und 48 in Eingriff treten. Gleichzeitig wird das proximale Ende der Betätigungsstange 36 über eine Kugelrastverbindung 50 mit dem beweglichen Handhabenteil 38 gekuppelt. Im Übrigen wird die Kupplung 26 mit dem Handhabenteil 3 in der aus DE 103 57 105 B3 bekannten Weise gekuppelt. An dem Handhabenteil 3 ist darüber hinaus eine Verdrehsicherung 52 in Form eines Vorsprunges vorgesehen, welcher in eine entsprechende Ausnehmung 53 an der Kupplung 26 eingreift und so das Außenrohr 8 drehfest mit dem Handhabenteil 3 in einer vorbestimmten Winkellage verbindet.

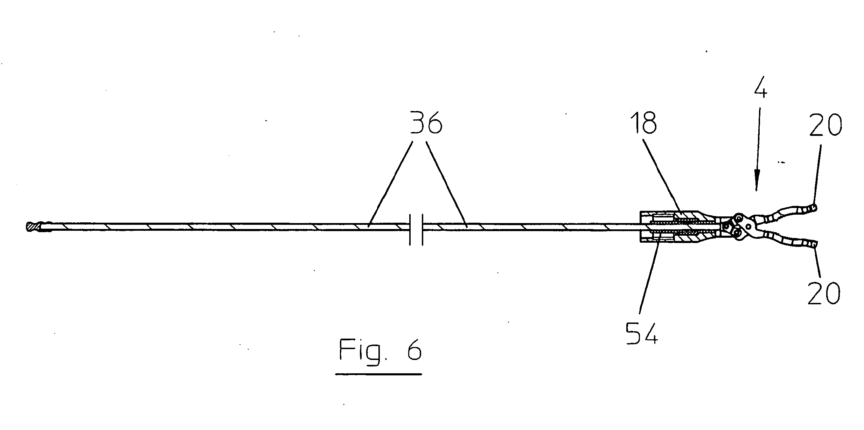

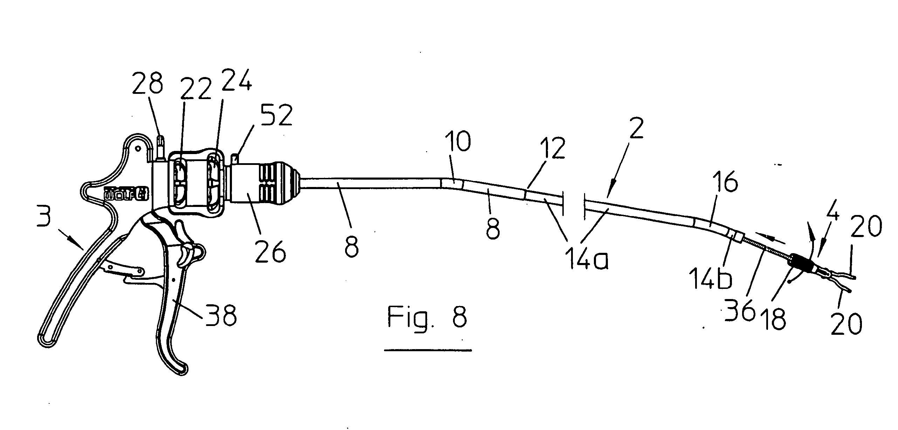

Fig. 6 zeigt das entnommene Maulteil 4, an welchem proximalseitig die Betätigungsstange 36 fest mit der Mechanik der beweglichen Teile 20 verbunden ist. Das Maulteil 4 kann mit der proximalen Gewindehülse 18 mit dem distalen Ende des äußeren Schaftrohres 14 verschraubt werden. Dabei tritt ein Kupplungsteil 54 formschlüssig mit dem distalen Ende des Innenrohres 34 in Eingriff, sodass eine Drehung von dem Innenrohr 34 über das Kupplungsteil 54 auf den distalen Abschnitt des Maulteils 4 möglich ist. In Fig. 8 ist das Maulteil 4 im teilweise entnommenen Zustand gezeigt.

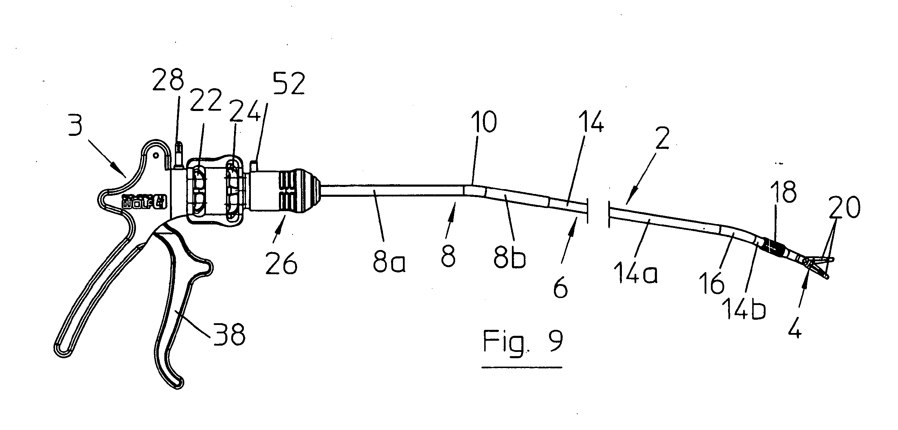

Fig. 9 zeigt noch einmal eine Seitenansicht des Instrumentes gemäß Fig. 1. Es ist zu verstehen, dass die Ausgestaltung des Maulteils 4 auf verschiedene Weise erfolgen kann. So kann auch die Ausgestaltung als Schere bei der zweiten Ausführungsform gemäß Fig. 2 oder umgekehrt die Ausgestaltung als Fasszange bei der ersten Ausführungsform gemäß Fig. 1 verwirklicht werden. Zur Verwendung des Instrumentes als HF-Instrument ist das äußere Schaftrohr 14 nach außen isoliert ausgebildet. Das Instrument muss jedoch nicht zwingend als HF-Instrument ausgebildet werden. In diesem Fall kann dann auf die Isolation sowie den HF-Anschluss 28 verzichtet werden.

### Bezugszeichenliste

- 1: - Instrument
- 2: - Zangeneinsatz
- 3: - Handhabenteil
- 4: - Maulteil
- 6: - Schaft
- 8, 8a, 8b: - Außenrohr
- 10: - Abwinkelbereich
- 12: - Distales Ende des Außenrohres
- 14, 14a, 14b: - Äußeres Schaftrohr
- 16: - Abwinkelbereich
- 18: - Gewindehülse
- 20: - Bewegliche Teile des Maulteils
- 22, 24: - Handräder
- 26: - Kupplung
- 28: - HF-Anschluss
- 30: - Abwinkelbereich
- 32: - Rohrabschnitt
- 34: - Innenrohr
- 35: - Rohrabschnitte
- 36: - Betätigungsstange
- 38: - Bewegliches Handhabenteil
- 40: - Rastsperre
- 42: - Vielkant
- 44: - Aufnahme
- 46: - Vielkant
- 48: - Aufnahme
- 40: - Kugelrastverbindung
- 52: - Verdrehsicherung
- 53: - Ausnehmung
- 54: - Kupplungsteil
- A, B, C -: Bewegungsrichtungen

## Patentansprüche

1. Medizinisches Instrument mit einer am proximalen Ende angeordneten Handhabe (3), einem sich ausgehend von der Handhabe (3) distalwärts erstreckenden Schaft (6) sowie einem am distalen Ende des Schaftes (6) angeordneten bewegbaren Maulteil (4), **dadurch gekennzeichnet, dass**
der Schaft (6) starr ausgebildet ist und zumindest einen proximalen (10) und einen distalen (16, 30) Abwinkelbereich aufweist, in welchen sich die Erstreckungsrichtung des Schaftes (6) ändert,
ein Abschnitt des Schaftes (6), in welchem der distale Abwinkeibereich (16, 30) gelegen ist, relativ zu einem Abschnitt des Schaftes (6), in dem der proximale Abwinkelbereich (10) gelegen ist, um die Längsachse drehbar ist,
und das Maulteil (4) relativ zu dem Schaft (6) um dessen Längsachse am distalen Ende drehbar ist.

2. Medizinisches Instrument nach Anspruch 1, **dadurch gekennzeichnet, dass** der Schaft (6) ein äußeres Schaftrohr (14) aufweist und der zumindest eine distale Abwinkelbereich (16, 30) durch einen Winkel oder eine Biegung in dem äußeren Schaftrohr (14) definiert ist.

3. Medizinisches Instrument nach Anspruch 2, **dadurch gekennzeichnet, dass** das äußere Schaftrohr (14) relativ zu einem proximalen Abschnitt des Schaftes (6) drehbar ist.

4. Medizinisches Instrument nach Anspruch 2 oder 3, **dadurch gekennzeichnet, dass** der Schaft (6) ein an die Handhabe (3) angrenzendes, sich nur über einen proximalen Abschnitt des Schaftes (6) erstreckendes starres Außenrohr (8) aufweist, welches das äußere Schaftrohr (14) außen umfänglich umgibt und den zumindest einen proximalen Abwinkelbereich (10) durch einen Winkel oder eine Biegung in dem Außenrohr (8) definiert.

5. Medizinisches Instrument nach Anspruch 3 und 4, **dadurch gekennzeichnet, dass** das äußere Schaftrohr (14) relativ zu dem Außenrohr (8) um seine Längsachse drehbar ist.

6. Medizinisches Instrument nach Anspruch 5, **dadurch gekennzeichnet, dass** äußere Schaftrohr (14) am proximalen Ende mit einer Dreheinrichtung (24) in der Handhabe (3) verbunden ist.

7. Medizinisches Instrument nach einem der Ansprüche 4 bis 6, **dadurch gekennzeichnet, dass** das äußere Schaftrohr (14) in dem proximalen Abwinkelbereich (10) biegsam, vorzugsweise aus mehreren beweglich miteinander verbundenen starren Rohrabschnitten (32), ausgebildet ist.

8. Medizinisches Instrument nach einem der Ansprüche 2 bis 7, **dadurch gekennzeichnet, dass** an dem distalen Ende des äußeren Schaftrohres (14) das Maulteil (4) drehbar angebracht ist und dass in dem äußeren Schaftrohr (14) ein drehbares Innenrohr (34) angeordnet ist, welches an seinem proximalen Ende mit einer Dreheinrichtung (22) in der Handhabe (3) und am distalen Ende drehfest mit dem Maulteil (4) verbunden ist.

9. Medizinisches Instrument nach Anspruch 8, **dadurch gekennzeichnet, dass** das Innenrohr (34) zumindest in den Abwinkelbereichen (10, 16, 30) biegsam, vorzugsweise aus mehreren beweglich miteinander verbundenen starren Rohrabschnitten (35), ausgebildet ist.

10. Medizinisches Instrument nach einem der vorangehenden Ansprüche, **dadurch gekennzeichnet, dass** sich im Inneren des Schaftes von der Handhabe (3) zu dem Maulteil (4) eine Betätigungsstange (36) zur Betätigung des Maulteils (4) erstreckt.

11. Medizinisches Instrument nach einem der vorangehenden Ansprüche, **dadurch gekennzeichnet, dass** das Maulteil (4) lösbar mit dem äußeren Schaftrohr (14) verbunden ist.

12. Medizinisches Instrument nach einem der vorangehenden Ansprüche, **dadurch gekennzeichnet, dass** der Schaft (6) lösbar mit der Handhabe (3) verbunden ist.

13. Medizinisches Instrument nach einem der vorangehenden Ansprüche, **dadurch gekennzeichnet, dass** das Maulteil (4) als Zange oder Schere ausgebildet ist.

14. Medizinisches Instrument nach einem der vorangehenden Ansprüche, **dadurch gekennzeichnet, dass** Instrument (1) als HF-Instrument mit nach außen isolierten Schaft (6) ausgebildet ist.

## Claims

1. A medical instrument with a handle (3) arranged at the proximal end, with a shank (6) extending distally proceeding from the handle (3), as well as a movable jaw part (4) arranged at the distal end of the shank (6),
**characterised in that**
the shank (6) is designed in a rigid manner and comprises at least one proximal (10) and one distal (16, 30) bend region, in which the extension direction of the shank (6) changes,
a section of the shank (6), in which the distal bend region (16, 30) is situated, may be rotated about the longitudinal axis relative to a section of the shank (6), in which the proximal bend region (10) is situated,
and the jaw part (4) is rotatable relative to the shank (6) about its distal longitudinal axis, at the distal end.

2. A medical instrument according to claim 1, **characterised in that** the shank (6) comprises an outer shank tube (14) and the at least one distal bend region (16, 30) is defined by an angle or a bend in the outer shank tube (14).

3. A medical instrument according to claim 2, **characterised in that** the outer shank tube (14) is rotatable relative to a proximal section of the shank (6).

4. A medical instrument according to claim 2 or 3, **characterised in that** the shank (6) comprises a rigid outer tube (8) which borders the handle (3), extends only over a proximal section of the shank (6), peripherally surrounds the outer shank tube (14) and defines the at least one proximal bend region (10) by an angle or a bend in the outer tube (8).

5. A medical instrument according to claim 3 and 4, **characterised in that** the outer shank tube (14) is rotatable about its longitudinal axis relative to the outer tube (8).

6. A medical instrument according to claim 5, **characterised in that** the outer shank tube (14) is connected at the proximal end to a rotation device (24) in the handle (3).

7. A medical instrument according to one of the claims 4 to 6, **characterised in that** the outer shank tube (14) in the proximal bend region (10) is flexible, preferably formed of several rigid tube sections (32) which are movably connected to one another.

8. A medical instrument according to one of the claim 2 to 7, **characterised in that** the jaw part (4) is rotatably attached on the distal end of the outer shank tube (14) and that a rotatable inner tube (34) is arranged in the outer shank tube (14), said rotatable inner tube being connected at its proximal end to a rotation device (22) in the handle (3), and at the distal end to the jaw part (4) in a rotationally fixed manner.

9. A medical instrument according to claim 8, **characterised in that** the inner tube (34) at least in the bend regions (10, 16, 30) is designed in a flexible manner, preferably of several rigid tube sections (35) which are movably connected to one another.

10. A medical instrument according to one of the preceding claims, **characterised in that** an actuation rod (36) for actuating the jaw part (4) extends in the inside of the shank from the handle (3) to the jaw part (4).

11. A medical instrument according to one of the preceding claims, **characterised in that** the jaw part (4) is releasably connected to the outer shank tube (14).

12. A medical instrument according to one of the preceding claims, **characterised in that** the shank (6) is releasably connected to the handle (3).

13. A medical instrument according to one of the preceding claims, **characterised in that** the jaw part (4) is designed as a forceps or as a scissors.

14. A medical instrument according to one of the preceding claims, **characterised in that** the instrument (1) is designed as a HF-instrument with a shank (6) which is insulated to the outside.

## Revendications

1. Instrument médical comprenant une poignée (3) disposée au niveau de l'extrémité proximale, une tige (6) qui s'étend dans la direction distale en partant de la poignée (3), ainsi qu'une pièce à mâchoires (4) mobile, disposée au niveau de l'extrémité distale de la tige (6), **caractérisé en ce que**
la tige (6) est conçue de façon rigide et présente au moins une zone coudée proximale (10) et une zone coudée distale (16, 30), dans lesquelles la direction d'extension de la tige (6) change,
un segment de la tige (6) dans lequel la zone coudée distale (16, 30) est logée peut tourner autour de l'axe longitudinal par rapport à un segment de la tige (6) dans lequel est logée la zone coudée proximale (10),
et la pièce à mâchoires (4) peut tourner autour de son axe longitudinal par rapport à la tige (6) au niveau de l'extrémité distale.

2. Instrument médical selon la revendication 1, **caractérisé en ce que** la tige (6) présente un tube extérieur de tige (14) et la zone coudée distale (16, 30), au moins au nombre de une, est définie par un angle ou une courbure du tube extérieur de tige (14).

3. Instrument médical selon la revendication 2, **caractérisé en ce que** le tube extérieur de tige (14) peut tourner par rapport à un segment proximal de la tige (6).

4. Instrument médical selon la revendication 2 ou 3, **caractérisé en ce que** la tige (6) présente un tube extérieur rigide (8), adjacent à la poignée (3) et qui s'étend seulement sur un segment proximal de la tige (6), qui entoure le tube extérieur de tige (14) sur la périphérie extérieure et définit la zone coudée proximale (10), au moins au nombre de une, par un angle ou une courbure du tube extérieur (8).

5. Instrument médical selon la revendication 3 et 4, **caractérisé en ce que** le tube extérieur de tige (14) peut tourner par rapport au tube extérieur (8) autour de son axe longitudinal.

6. Instrument médical selon la revendication 5, **caractérisé en ce qu'**au niveau de l'extrémité proximale, le tube extérieur de tige (14) est relié à un dispositif de rotation (24) contenu dans la poignée (3).

7. Instrument médical selon l'une des revendications 4 à 6, **caractérisé en ce que**, dans la zone coudée proximale (10), le tube extérieur de tige (14) est conçu de façon flexible, de préférence composé de plusieurs segments de tube rigides (32) reliés les uns aux autres de façon mobile.

8. Instrument médical selon l'une des revendications 2 à 7, **caractérisé en ce que** la pièce à mâchoires (4) est appliquée au niveau de l'extrémité distale du tube extérieur de tige (14) de façon à pouvoir tourner, et **en ce que** dans le tube extérieur de tige (14) est disposé un tube intérieur (34) pouvant tourner, lequel, à son extrémité proximale, est relié à un dispositif de rotation (22) placé dans la poignée (3) et qui, à l'extrémité distale, est relié solidairement en rotation à la pièce à mâchoires (4)..

9. Instrument médical selon la revendication 8, **caractérisé en ce qu'**au moins dans les zones coudées (10, 16, 30), le tube intérieur (34) est conçu de façon flexible, de préférence composé de plusieurs segments de tube rigides (35) reliés les uns aux autres de façon mobile.

10. Instrument médical selon l'une des revendications précédentes, **caractérisé en ce qu'**une tige de commande (36) servant à actionner la pièce à mâchoires (4) s'étend à l'intérieur de la tige, de la poignée (3) jusqu'à la pièce à mâchoires (4).

11. Instrument médical selon l'une des revendications précédentes, **caractérisé en ce que** la pièce à mâchoires (4) est reliée au tube extérieur de tige (14) de façon démontable.

12. Instrument médical selon l'une des revendications précédentes, **caractérisé en ce que** la tige (6) est reliée à la poignée (3) de façon démontable.

13. Instrument médical selon l'une des revendications précédentes, **caractérisé en ce que** la pièce à mâchoires (4) est conçue en tant que pince ou ciseaux.

14. Instrument médical selon l'une des revendications précédentes, **caractérisé en ce que** l'instrument (1) est conçu en tant qu'instrument HF dont la tige (6) est isolée extérieurement.
